# EUROPEAN PATENT APPLICATION

(11) **EP 0 622 461 A2**
(43) Date of publication of application: **02.11.1994**
(21) Application number: 94104884.5
(22) Date of filing: 28.03.1994
(51) Int. Cl.: C12N 15/82, C12N 15/40, A01H 5/00

(54) **Preparation of yellow mosaic disease-resistant barley**

(30) Priority: 29.03.1993 JP 91933/93
(71) Applicant: DIRECTOR OF NATIONAL AGRICULTURE RESEARCH CENTER, MINSTERY OF AGRICULTURE, FORESTRY AND FISHERIES, Tsukuba-shi, Ibaraki-ken (JP); SAPPORO BREWERIES LIMITED, Tokyo (JP)
(72) Inventor: Kuroda, Hisao, c/o Sapporo Breweries Ltd., Nitta-cho, Nitta-gun, Gunma-ken, (JP); Hirota, Naohiko, c/o Sapporo Breweries Ltd., Nitta-cho, Nitta-gun, Gunma-ken, (JP); Ito, Kazutoshi, c/o Sapporo Breweries Ltd., Nitta-cho, Nitta-gun, Gunma-ken, (JP); Kashiwazaki, Satoshi, Tsukuba-shi, Ibaraki-ken (JP); Hibino, Hiroyuki, Tsukuba-shi, Ibaraki-ken (JP)
(74) Representative: VOSSIUS & PARTNER

(57) **Abstract**

The present invention provides a novel method of preparing a yellow mosaic disease-resistant barley without requiring long-term breeding or having significant adverse effects on essential characters of barley due to introduction of a resistance to yellow mosaic disease-causing viruses, that is, a barley yellow mosaic virus (BaYMV) and a barley mild mosaic virus (BaMMV). The method of the invention includes the steps of: (a) isolating a gene including a specific gene region coding a capsid protein, as a capsid protein gene, from cDNA of a barley yellow mosaic virus genome or a barley mild mosaic virus genome; (b) conjugating the capsid protein gene with a plasmid vector or rearranging the capsid protein gene and then conjugating the rearranged gene with a plasmid vector, in order to construct a vector DNA having an ability to transform barley cells; (c) transforming the barley cells with the vector DNA; and (d) making the transformed barley cells regenerated in a barley plant, so as to make the capsid protein originated from the capsid protein gene expressed in the barley plant to prepare the yellow mosaic disease-resistant barley. The method of the invention is applicable to different strains of both the BaYMV and BaMMV.

## Description

The present invention relates to a method of preparing a yellow mosaic disease-resistant barley, and more specifically to the preparation of a yellow mosaic disease-resistant barley by isolating a specific gene region coding a capsid protein of a barley yellow mosaic virus (hereinafter referred to as BaYMV) or a barley mild mosaic virus (hereinafter referred to as BaMMV), recombining the gene region as a capsid protein gene, and making the recombined gene to be expressed in barley plants.

Development of a new cultivar of barley with sufficient resistance to the barley yellow mosaic virus is of great importance especially in Japan, China, Europe, and other yellow mosaic disease-tainted regions. The yellow mosaic disease-resistant barley has conventionally been bred by introducing a specific resistant gene of a barley species having a resistance to BaYMV through a long-term mating process.

There are three different generally-known strains of BaYMV, that is, types I, II, and III. Giving a barley breed sufficient resistance to all three strains of BaYMV has not yet been successfully achieved. It is quite difficult to introduce a plurality of resistant genes by a conventional mating technique without having significant adverse effects on essential characters of barley. Recent study on the BaMMV has also revealed the spread of BaMMV-caused yellow mosaic.

One object of the present invention is thus to provide a novel method of preparing a yellow mosaic disease-resistant barley.

Another object of the invention is to provide a method for the preparation of a yellow mosaic disease-resistant barley without requiring long-term breeding or having significant adverse effects on essential characteristics of barley due to the introduction of resistance to yellow mosaic disease-causing viruses.

As described previously, two different viruses BaYMV and BaMMV, each having a plurality of strains, cause yellow mosaic disease in barley.

The inventors have clarified gene structures of two different yellow mosaic disease-causing viruses, BaYMV and BaMMV, through a long-term study of the molecular biology of yellow mosaic disease in barley, and specified gene regions coding capsid proteins particularly concerned with the invention. These results are presented in Kashiwazaki et al., Journal of General Virology, 70, 3015-3023(1989), Kashiwazaki et al., Journal of General Virology, 73, 2173-2181(1992), and Japanese Patent Kokai Hei 4-180624. Based on these results, the inventors have completed a method of preparing a yellow mosaic disease-resistant barley by isolating a gene region coding a capsid protein of BaYMV or BaMMV, recombining the gene region as a capsid protein gene, and introducing the recombined gene into barley cells to make the recombined gene expressed in the barley cells so as to produce the capsid protein and give resistance to the yellow mosaic disease-causing viruses.

The invention provides a method of preparing a yellow mosaic disease-resistant barley. The method includes the steps of:
(a) isolating a gene including a specific gene region coding a capsid protein, as a capsid protein gene, from cDNA of a barley yellow mosaic virus genome;
(b) conjugating the capsid protein gene with a plasmid vector or recombining the capsid protein gene and then conjugating the recombined gene with a plasmid vector, in order to construct a vector DNA having the ability to transform the barley cells;
(c) transforming the barley cells with the vector DNA; and
(d) making the transformed barley cells regenerate in a barley plant, so that the capsid protein originates from the capsid protein gene expressed in the barley plant to prepare the yellow mosaic disease-resistant barley.

Alternatively, the method includes the steps of:
(a) isolating a gene including a specific gene region coding a capsid protein, as a capsid protein gene, from cDNA of a barley mild mosaic virus genome;
(b) conjugating the capsid protein gene with a plasmid vector or recombining the capsid protein gene and then conjugating the reassorted gene with a plasmid vector, in order to construct a vector DNA having the ability to transform barley cells;
(c) transforming the barley cells with the vector DNA; and
(d) making the transformed barley cells regenerate in a barley plant, so that the capsid protein originates from the capsid protein gene expressed in the barley plant to prepare the yellow mosaic disease-resistant barley.

The method of the invention does not require long-term breeding and has substantially no adverse effects on essential characteristics of barley, unlike the conventional mating technique for development of yellow mosaic disease-resistant barley breed. The method of the invention is applicable to different strains of both the BaYMV and BaMMV.

These and other objects, features, aspects, and advantages of the present invention will become more apparent from the following detailed description of the preferred embodiment of this invention with the accompanying drawings.
Fig. 1 shows the construction of plasmids making capsid protein genes expressed in Escherichia coli;
Figs. 2A and 2B schematically show electrophoretic migration of capsid proteins produced in Escherichia coli;
Fig. 3 shows the construction of plasmids making capsid protein genes expressed in barley cells; and
Figs. 4A and 4B schematically show the electrophoretic migration of capsid proteins produced in barley cells.

More specifically, BaYMV and BaMMV-originated capsid protein genes and the yellow mosaic disease-resistant barley having such genes are prepared according to steps explained below.

Preparation of a BaMMV-originated capsid protein gene DNA is first explained.

### Isolation of BaMMV

The BaMMV is isolated by fractional centrifugation. sucrose gradient centrifugation, or cesium chloride gradient centrifugation generally used for the concentration and purification of plant viruses.

According to a typical method of isolating the BaMMV proposed by Usugi, Saitoh, et al, (Annals of the Phytopathological Society of Japan, Vol. 42, 1, 1976), barley mild mosaic virus (BaMMV)-tainted leaves are ground in a buffer, for example, a 0.1M citrate buffer or a 0.1M phosphate buffer, which is then filtered through a gauze to a crude solution. After treating the crude solution with an organic solvent, such as carbon tetrachloride or ethyl ether, the target virus BaMMV is separated from the residue and concentrated to give a concentrated virus preparation through repeated high-speed centrifugation (5,000 through 10,000 x g) and ultracentrifugation (80,000 through 100,000 x g). The concentrated virus preparation thus obtained is further treated by sucrose gradient centrifugation or cesium chloride gradient centrifugation, and a virus phase of the centrifuged preparation is finally separated and desalted to give a purified virus preparation.

An Na1 strain or a Ka1 strain of the BaMMV is isolated from the barley mild mosaic virus-tainted leaves in the above manner (see Kashiwazaki S. et al., 'Proceedings of the First Symposium of the International Working Group on Plant Viruses with Fungal Vectors, Braunschweig 105-108 (1990)).

### Separation and Identification of BaMMV Na1 or Ka1 Strain

In a preferred embodiment, an Na1 strain and a Ka1 strain of the BaMMV were respectively collected from yellow mosaic-tainted leaves found in Japan in Natajima, Yamaguchi prefecture and Kagawa, and subcultured and purified through inoculation. Both the Na1 strain and the Ka1 strain are stored in a plant virus laboratory, the National Agricultural Research Center, the Ministry of Agriculture, Forestry and Fisheries.

An antibody was prepared to each purified virus strain. Na1 and Ka1 strains of BaMMV were then identified through cross-reaction of the antibody with the purified BaYMV or BaMMV strains (see Kashiwazaki S. et al., 'Proceedings of the First Symposium of the International Working Group on Plant Viruses with Fungal Vectors, Braunschweig 105-108 (1990)).

### Extraction of RNA from BaMMV

RNA is extracted from BaMMV according to any of the known methods, such as the guanidine method, the thermal phenol method or the SDS-phenol method.

### Synthesis of cDNA from RNA

According to a conventional method, for example, the Okayama and Berg method, the Shovel method, the Gubler and Hoffman method, the S1 nuclease method, or the Land's method, cDNA is prepared by treating the RNA of BaMMV thus extracted.

### Cloning of cDNA

The cDNA synthesized as above is integrated into a plasmid vector or lambda phage vector directly or through ligation with an adaptor or a linker DNA.

### Determination of Base Sequence

The base sequence of the cDNA-cloned plasmid is determined according to the Maxam-Gilbert method or dideoxyribonucleotide sequencing.

According to the steps described above, DNA containing a gene coding a capsid protein of the BaMMV is prepared and identified.

### Isolation of BaMMV Capsid Protein

The ratio of a capsid protein to the total content of BaMMV-structual proteins including a genome binding protein and a capsid protein is not less than 99%. The capsid protein is purified by means of sodium dodecyl sulfate polyacrylamide gel electrophoresis (SDS-PAGE) or high-performance liquid chromatography (HPLC).

### Determination of N-terminal Amino-Acid Sequence in BaMMV Capsid Protein

The N-terminal amino-acid sequence in a protein is generally determined by an enzyme method which successively eliminates amino-acids from the N-terminal with peptidase or by a degradation method which marks N-terminal amino-acids and identifies the marked amino-acids. The Edman degradation method using phenylisothiocyanate, a determination method of the latter type, is applied most commonly. A device for automatically determining the amino-acid sequence using the Edman degradation method is commercially available from Applied Biosystems (ABI Co., Ltd.)

In the preferred embodiment, the N-terminal amino-acid sequence of the BaMMV capsid protein isolated and purified by means of SDS-PAGE was determined with a protein sequencer (ABI Co., Ltd).

The primary structure or amino-acid sequence of the BaMMV capsid protein was determined by comparing the N-terminal amino-acid sequence of the BaMMV capsid protein thus determined with the base sequence of the BaMMV gene.

A complementary base sequence of the BaMMV capsid protein gene thus determined is prepared by chemical synthesis or genetic engineering, and hybridized under such conditions as given below to obtain identification without using a conventional immunological technique. The BaMMV caps id protein gene isolated under the hybridization conditions is an embodiment of the BaMMV capsid protein DNA applicable to the invention. A BaYMV capsid protein gene is obtained in a manner similar to that for the BaMMV.

### Preparation of Plasmid with cDNA including BaYMV and BaMMV Capsid Protein Genes

A method of preparing cDNA from BaYMV and MaMMV genomes and subcloning a specific region coding the capsid proteins has already been presented by the inventors in Kashiwazaki et al., Journal of General Virology, 70, 3015-3023(1989), Kashiwazaki et al., Journal of General Virology, 73, 2173-2181(1992), and a specification of Japanese Patent Kokai Hei 4-180624. In the preferred embodiment, plasmids pBYT10, Ka1SK11, and Na1SK2, each having cDNA including specific regions coding BaYMV and BaMMV capsid protein genes, were prepared according to the proposed method.

### Subcloning of Specific Region Which Codes Capsid Protein and Recombination of Outercoat Protein Gene

Typical methods of subcloning a gene include digestion of a target region with an appropriate restriction enzyme and gene amplification with DNA polymerase or a polymerase chain reaction (hereinafter referred to as PCR). Requiring that a specific region code the capsid protein should function as an independent gene, the inventors have applied the polymerase chain reaction allowing the terminal structure to be freely designed. Primer DNAs required for the PCR are generally synthesized with a commercially available DNA synthesizer. In the embodiment, the primer DNAs were synthesized with an Applied Biosystems 391 DNA synthesizer, PCR-MATETM EP (ABI Co., Ltd.) utilizing the phosphoamidide method.

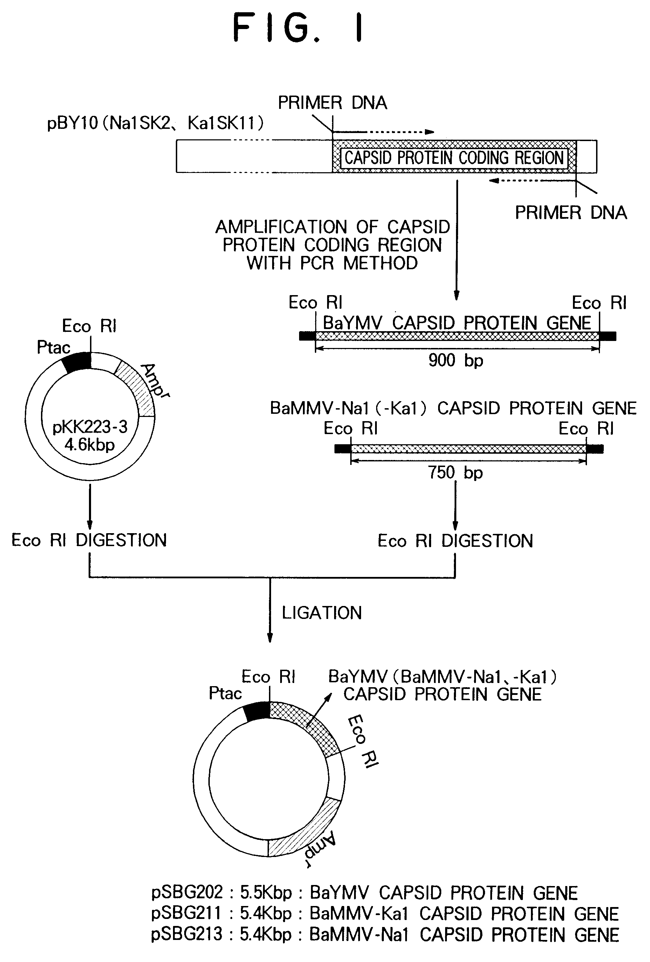

The primer DNA thus synthesized may be purified using liquid chromatography or an oligonucleotide purification column. In the preferred embodiment, the primer DNA with a trityl group added thereto during synthesis of the DNA was purified according to a predetermined method with an oligonucleotide purification column commercially available from Applied Biosystems Co., Ltd. Using an EcoRI-digested plasmid including the capsid protein gene as a template, the capsid protein genes of BaYMV and BaMMV were amplified according to the polymerase chain reaction with the synthesized primer DNA. The amplified sample was then extracted with phenol, digested with EcoRI, and fractionated through agarose gel electrophoresis for isolation of a DNA band corresponding to the capsid protein gene. The DNA segment was subsequently inserted into a site of EcoRI incision on an Escherichia coli-expressing vector, pKK223-3, so that recombinant plasmids pSBG202, pSBG211, and pSBG213, each having the capsid protein gene, were constructed. Fig. 1 shows construction of such plasmids making capsid protein genes expressed in Escherichia coli.

Production of Capsid Protein in Escherichia coli

The target function of a DNA segments cloned or artificially constructed is evaluated by the production of a target protein originated by the gene. In the preferred embodiment, it was determined whether each virus capsid protein gene subcloned and recombined in the above manner could function as a gene producing the capsid protein by assaying preparation of the capsid protein in Escherichia coli. Addition of isopropyl-β-D-galactopyranoside (hereinafter referred to as IPTG) to a medium activates a tac promoter of the Escherichia coli-expressing vector pKK223-3 to make the inserted gene to be expressed therein. Insertion of a capsid protein gene into an EcoRI site of the pKK223-3 allows a ribosome-binding sequence on the vector to be utilized for the translation of the gene.

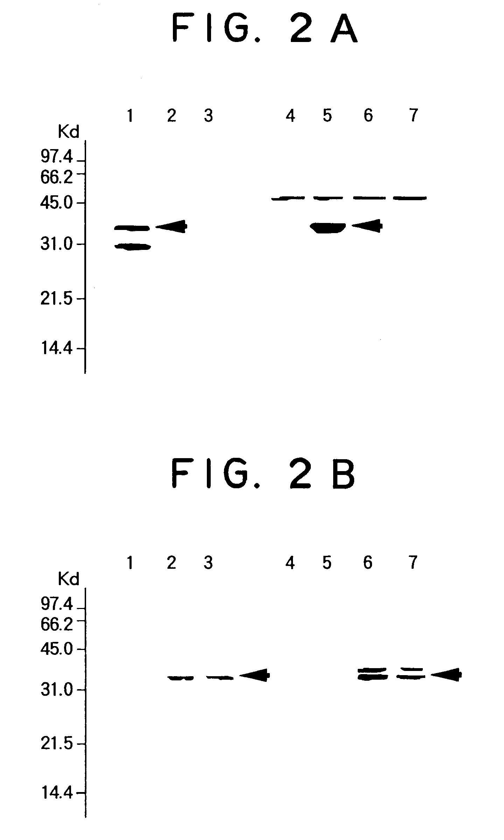

In the preferred embodiment, XL-1 strains of transformed Escherichia coli were cultivated. The transformed E. coli had a capsid protein gene-containing recombinant plasmid pSBG202, pSBG211, or pSBG213, or a control pKK223-3. Each vector was assayed for the ability of producing the corresponding capsid protein by inducing expression of the target gene through addition of IPTG in the middle of logarithmic multiplication. Total proteins in E. coli treated for the expression of the target gene were fractionated through SDS-polyacrylamide electrophoresis, and blotted onto a PVDF (polyvinylidene difluoride) membrane, Immobilon P (Millipore Corp.) by western blotting to be reacted with an antibody of a target capsid protein. This allows the target capsid protein to be effectively detected among the total proteins. The protein thus detected was originated from the capsid protein gene and had a molecular weight identical to that of the virus-originated capsid protein. This clearly shows that the capsid protein gene constructed as above has the ability to produce the corresponding capsid protein (see Figs. 2A and 2B).

### Production of Capsid Protein in Barley Cells

For the production of yellow mosaic disease-resistant barley with an appropriate capsid protein gene, it is necessary to make the capsid protein gene to be expressed in barley cells to produce the corresponding capsid protein. A promoter and a transcription terminating factor functioning in plant cells are essential for the expression in plant cells of the capsid protein gene assayed as previously described. Preferable promoters include a generally known 35S promoter of a cauliflower mosaic virus, or other gene promoters specifically and efficiently expressed in roots or leaves since the barley yellow mosaic virus infects barley from the roots and develops the yellow mosaic in leaves. In the embodiment, the inventors used the 35S promoter and a transcription terminating sequence of nopaline synthase (hereinafter referred to as NOS).

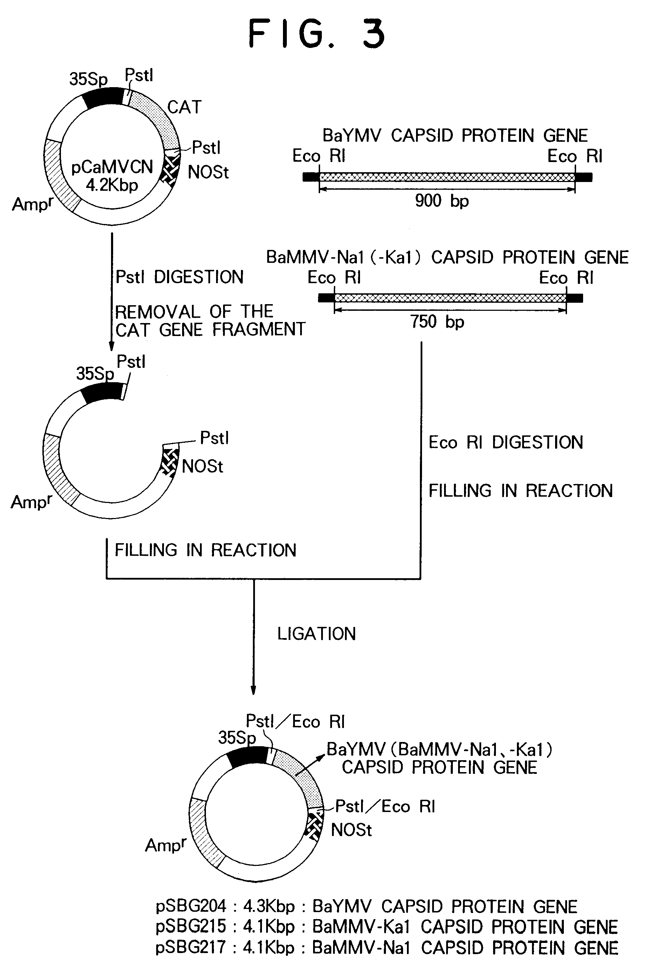

A commercially available control vector pCaMVCN (Pharmacia Corp.) used for direct gene transduction in to plant cells utilizes the 35S promoter and the NOS transcription terminating factor to allow chloramphenicol acetyltransferase (hereinafter referred to as CAT) gene to be expressed in plant cells. In the preferred embodiment, plasmids pSBG204, pSBG215, and pSBG217 to allow expression of each capsid protein gene were constructed by replacing the CAT gene in the plasmid pCaMVCN with the capsid protein gene (see Fig. 3). Such a recombinant plasmid is directly transduced into barley cells by electroporation, laser processing, particle gun processing, or polyethylene glycol treatment.

In the embodiment, electroporation was applied to transduction of the target gene into barley cells. Barley cells used herein were Hordeum vulgare immature embryo-originated R215S cells. A protoplast of the R215S cells was prepared according to a conventional method of preparation using a cellulolytic enzyme and a pectolytic enzyme. A target gene and a hygromycin-resistant gene used as a selective marker were simultaneously transduced into the protoplast. Barley cells with the target capsid protein gene transduced therein were obtained according to a method of co-transformation selecting hygromycin-resistant cells (Shimamoto et al., Nature, 338, 274-276(1989)).

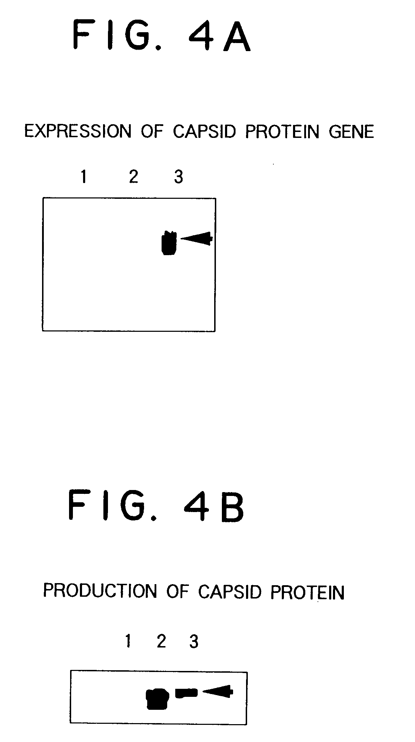

The plasmids pSBG204, pSBG215, and pSBG217 having a hygromycin-resistant gene pSBG102 (Kaneko et al., Barley Genetics VI volume I, pp.231-233(1991)), and a control pCaMVCN were transduced into the protoplast with Gene Pulser (Biorad Inc). The protoplast with the gene transduced thereinto was cultured in a medium containing hygromycin, and hygromycin-resistant cells were selected from the culture. Cells with each capsid protein gene transduced thereinto were identified from among the hygromycin-resistant cells by southern hybridization. Expression of the capsid protein gene in the cultured cells was assayed by northern hybridization for detecting a transcript mRNA (Alwine, J.C., et al. Proc. Natl. Acad. Sci. 74:5350(1977)) or by detection with an antibody to the target capsid protein as described above. Results of the experiment suggest that the production of a transcript or capsid protein originated from the transduced capsid protein gene (see Figs. 4A and 4B). This method is thereby applicable to the production of yellow mosaic disease-resistant barley.

### Production of Yellow Mosaic Disease-resistant Barley

There are several methods applicable for the transduction of a target gene into barley cells to produce transformed barley. One preferable method is disclosed in 'Production of Transformed Barley with Laser' Japanese Patent Kokai Hei 2-054607 by the inventors of the present invention.

In the proposed method, a target gene is transduced into pollen cells included in anthers of barley, which have apertures previously formed with a laser pulse, so that gene information in the target gene is expressed in barley. The pollen cells used are early pollen cells or spherical cell blocks before the rupture of pollen sacs in the mononuclear metaphase.

The method applied to the invention is described in more detail. The pollen cells as described above are suspended into a solution, typically an aqueous solution, containing a target gene having gene information to be transduced. A laser emitted from a laser system is focused on one of the pollen cells to form an aperture in the cell wall, through which the target gene is transduced into the pollen cell. The pollen cells are cultured in a callus-inducing medium to form calluses and/or germ layers, which are placed in a regeneration medium for the production of transformed barley.

Anthers of barley are cultivated in a callus-inducing medium for culturing pollen cells included in the anthers at a stage immediately before the division of the pollen cells in the mononuclear metaphase or an initial stage of such division. An appropriate culture medium is selected according to the characteristics of the anthers to be cultivated. Typical examples of such media include a modified MS medium (Carlsberg Res. Commun. Vol. 52, 393(1987)), an FHG medium (Kasha et al., XIX Stadler Genetics Symp. 213(1989)), Clapham I, II, III media (Z. Pflansenzucht, Vol. 69, 142(1973)), a Foroughi-Weir medium (Z. Pflanzenzucht, Vol. 77, 198(1976)), or modified media thereof. Conditions of cultivation are varied according to the anthers. The temperature of cultivation is generally 22 through 28°C, or more preferably around 25°C. The appropriate pH for cultivation is 5.6 through 6.0, or more specifically 5.8. The cultivation term is 0 through 14 days in general. The pollen cells are processed with a laser after being divided to have the ability of replicating DNA in the callus-inducing medium, that is, being transformed to cytoplasm-rich cells. The pollen cells are generally transformed to such cytoplasm-rich cells suitable for laser processing within two weeks.

Alternatively, pollen cells may be prepared by the method of Ziauddin et al. (Plant Cell Reports, Vol. 9, 59(1990)).

The barley to be used here may be Dissa, Igri, TRIUMPF, CARINA, or Haruna Nijo. Anthers are collected from the barley according to a conventional method.

The pollen cells preferably used are those in mononuclear metaphase isolated from the anthers.

The solution contains a 10 to 20,000 µl/ml target gene used for transduction into the pollen cells and other required constituents, that is, inactive salts for accelerating the balance or hypertonicity of the exact osmotic pressure, nutrients, and other additives. A preferable example of such solution is a gene suspension containing from 9 to 15% mannitol.

A laser emitted from a laser system was focused on one of the pollen cells to form an aperture in the cell wall, through which the target gene is transduced into the pollen cell as described above. The size of the aperture may be varied according to the requirements but should not be excessively large for the cell. The aperture generally has a diameter ranging from 5 through 500 nm. Application of the pulse continues for from 5 to 20 nano seconds, or more preferable 10 through 15 nano seconds. The pulse energy is adjusted generally in a range of from 0.1 to 10 microjoules. The laser system used herein may be any device which allows a laser to be focused onto a very small point. A preferable example is a Hitachi Laser Cell Processor (Hitachi, Ltd.) applicable to laser microinjection of mammalian cells.

After the laser process, the pollen cells are incubated in the solution containing the target gene for a time period sufficient for diffusion and invasion of the gene into the pollen cells with apertures. The incubation time is generally from 5 seconds to 2 hours, and the incubation temperature ranges from 0 °C to 28 °C.
After completion of the laser process and incubation described above, the pollen cells or cells induced therefrom are cultured to give a plant body. These procedures may follow the method of Olsen et al. (Carlsberg Res. Commun. Vol. 52, 393(1987)) or the method of Ziauddin et al. (Plant Cell Reports, Vol. 9, 59(1990)). Preferably, nurse cells used in application.

The method of transformation described above produces transformed barley having the corresponding virus capsid protein gene. The capsid protein gene thus transduced allows a capsid protein to be produced in barley cells to give sufficient resistance to the barley yellow mosaic virus or the barley mild mosaic virus.

### 1. Purification of Na1 and Ka1 Strains of BaMMV

After 270 ml of an icy-cold 0.1M citrate buffer (pH 6.8) were added to 100g of BaMMV-tainted leaves placed in an icy-cold mortar, the mixture was sufficiently ground. All the procedures were performed at a temperature of 4°C unless otherwise specified. A total of 300 g BaMMV-tainted leaves was treated through repetition of the above procedures. The ground solution was filtered through a gauze and sufficiently stirred with one fifth of the volumes of carbon tetrachloride and ether. A supernatant of the ground solution was obtained through centrifugation at of 1,000 x g and 4°C for ten minutes. The supernatant was further centrifuged at 5,000 x g and 4°C for fifteen minutes. A supernatant obtained by the second centrifugation was then ultracentrifuged at of 80,000 x g and 4°C for one hour to give a precipitation.

The precipitation thus obtained was resuspended in a 0.1M citrate buffer and centrifuged three times at 5,000 x g for ten minutes and at 100,000 x g for one hour to yield virus particles. The virus particles were suspended in 4 ml of a 0.1M citrate buffer containing 1.33g of CsCl and ultracentrifuged at 150,000 x g for sixteen hours. A virus phase of the centrifuged preparation was recovered with a syringe. The collected virus phase was suspended in a 0.1M citrate buffer and centrifuged at 55,000 x g for one hour to give a precipitation of virus particles. The precipitation of virus particles thus obtained was resuspended in the 0.1M citrate buffer and stored as purified virus stock.

### 2. Purification of RNA Extracted from Na1 and Ka1 Strains of BaMMV

RNA was extracted respectively from the purified Na1 and Ka1 strains of BaMMV according to the SDS phenol method.

A precipitation of viruses was recovered by centrifuging 0.8ml of the BaMMV Na1 stock suspension (O.D. 260=4.1) at 500,000 x g for twenty minutes. After the precipitation was suspended in 500 µl of a TE buffer (10 mM Tris-HCl, 1 mM EDTA both at pH 8.0), the suspension was mixed and stirred with 10 µl of 20% DSD and 20 µl of a 20 mg/ml aqueous solution of Protease K (Boehringer Corp.) and then incubated at 37°C for twenty minutes.

The suspension was then extracted twice with a phenol-chloroform (1:1) solution. After extraction of a water phase with chloroform (twice) and ether (three times), the water phase was mixed with 40 µl of 3M sodium acetate (pH 4.8), 2 µl of a 10 mg/ml aqueous solution of glycogen (Boehringer. Corp.), and 1 ml of ethanol and stood at -80°C for twenty minutes. The mixed solution was centrifuged at 15,000 x g for five minutes to give a precipitation. The precipitation thus obtained was centrifugally washed twice with 70% ethanol. After removal of ethanol, the precipitation was dissolved in 20 µl of distilled water and stored as RNA fractions.

### 3. Synthesis of BaMMV Na1 cDNA and BaMMV Ka1 cDNA

With a cDNA Synthesis System Plus (Amersham Co., Ltd.), cDNA was synthesized by treating an RNA fraction isolated and purified in the above manner. A mixture of 6 µl of an RNA solution (0.9 µg/µl of a TE buffer) and 16.5 µl of distilled and autoclaved water was heated at 65°C for two minutes and then cooled with ice. According to a protocol of Amersham, cDNA was synthesized. The cDNA solution obtained was made to flow in a mini-column packed with Sepharose 2B for removal of non-target proteins and low-molecular substances and yield of purified cDNA fractions.

### 4. Cloning of BaMMV Na1 cDNA and BaMMV Ka1 cDNA to Plasmid pBluescriptII

(1) For the cloning of BaMMV Na1 cDNA and BaMMV Ka1 cDNA to a plasmid pBluescriptII, ligation and size fractionation of an adaptor was performed. A purified cDNA fraction obtained in the above manner was precipitated with ethanol, dried, and dissolved in 5 µl of distilled water. The dissolved cDNA solution was mixed with 1 µl of a 10 pmol/µl EcoRI-NotI-BamHI adapter (Takara Shuzo Co., Ltd.) and 48 µl of a solution A and 6 µl of a solution B of a DNA ligation kit (Takara Shuzo Co., Ltd.), and ligated at 16 °C for two hours.
   The reaction mixture was precipitated with ethanol, dried, and dissolved in 5 µl of a TE buffer. A gel containing cDNA not less than 2 kb was incised through electrophoresis with a low-melting-point agarose, SeaKemK GTG Agarose (FMC Co., Ltd.), and placed in an Eppendolf tube. The gel with 100 µl of TE buffer was heated and melted at 65°C for five minutes, treated with phenol, precipitated in ethanol, and dissolved in 10 µl of distilled water.
(2) For cDNA of the BaMMV Na1 strain, 5 µl of the sample solution obtained in step (1) was mixed with 5 µl of 10 mM ATP, 5 µl of a 10x phosphorylating buffer (containing 660 mM of Tris-HCl buffer (pH 7.6), 66 mM of MgCl₂, 100 mM of dithiothreitol, 1 mM spermidine), 1 µl of T4 Polynucleotide kinase, and 34 µl of distilled water, and incubated at 37°C for one hour for phosphorylation. The reaction mixture was extracted with phenol, precipitated with ethanol, and dissolved in 5 µl of distilled water.
   The sample solution thus treated was then mixed with 1 µl of a 0.02 µg/µl plasmid pBluescript SK(+) (Stratagene Co., Ltd.) digested with EcoRI and dephosphorylated, and 48 µl of a solution A and 6 µl of a solution B of a DNA ligation kit (Takara Shuzo Co., Ltd.), and ligated at 16°C for two hours.
   For cDNA of the BaMMV Ka1 strain, the sample solution obtained in step (1) was mixed with 1 µl of a 0.02 µg/µl plasmid pBluescript SK(+) (Stratagene Co., Ltd.) digested with SmaI and dephosphorylated, and 48 µl of a solution A and 6 µl of a solution B of a DNA ligation kit (Takara Shuzo Co., Ltd.), and ligated at 16°C for two hours.
(3) The reaction solution obtained in step (2) was mixed on ice with 200 µl of competent cells of Escherichia coli NM522 (Stratagene Co., Ltd.) previously prepared, and left for thirty minutes. After being incubated at 42°C for two minutes, the solution was immediately cooled with ice, and incubated with 0.9 ml of an SOC culture medium at 37°C. After one hour, the incubated solution was spread out on ten plates of an LB agar medium (9 cm in diameter) containing 150 µg /ml 0.02% 5-bromo-4-chloro-3-indoryl-β-D-galactopyranoside and 0.5 mM isopropylthiogalactoside, and cultured overnight at 37°C. A plasmid DNA was extracted from white colony-originated Escherichia coli, and Escherichia coli having a long cDNA fragment-inserted plasmid was chosen and stored.
(4) RNA1-originated cDNA was prepared in the following manner. RNA extracted from the BaMMV Na1 and Ka1 strains was purified according to the method described in step (2). A gel containing an RNA1 band was incised through electrophoresis with a low-melting-point agarose, SeaKem GTG Agarose (FMC Co., Ltd.), and placed in an Eppendolf tube. The gel with 100 µl of TE buffer was heated and melted at 65°C for five minutes, extracted with phenol, precipitated with ethanol, and dissolved in 10 µl of distilled water. The RNA solution thus prepared was treated with an ECL system (Amasham Co., Ltd.) for dot hybridization using the cDNA clone obtained in step (3) as a probe.

As a result of the dot hybridization, obtained were strains of Escherichia coli having plasmids Na1SK2, Na1SK15, Ka1SK11, and Ka1SK46 with RNA1-originated cDNA segments inserted therein. The plasmids of the strains had cloned cDNA segments of 3.5 Kb, 3.5 Kb, 4Kb, and 3.8 Kb, respectively.

### 5. Determination of Base Sequence of BaMMV Na1 genome RNA-originated and BaMMV Ka1 genome RNA-originated cDNA

An overnight culture of Escherichia coli NM522 having the plasmids Na1SK2, Na1SK15, Ka1SK11, and Ka1SK46 was laid on 3 ml of a 2xYT medium (pH 7.2) containing 16 g/l bacto-trypton, 10g/l yeast extract, and 10g/l Nacl, and incubated at 37°C. The culture with a helper phage R408 added to m.o.i. 20 was further incubated overnight. A single-stranded DNA was purified after separation of the phage according to a known method. Using the single-stranded DNA as a template, the base sequence of cDNA was determined using the dideoxyribonucleotide sequencing method with a DNA sequencer 373A (ABI Co., Ltd).

### 6. Determination of N-terminal Amino-acid Sequence of Capsid Protein

The capsid protein separated from 10 µl of a solution containing a BaMMV Na1 strain or BaMMV Ka1 strain (1.5 mg/ml 0.1M citrate buffer: pH 6.8) through SDS-PAGE was transcribed on a membrane ProBlott (ABI Co., Ltd.) using western blotting, and dyed with Coomassie brilliant blue. A band corresponding to the capsid protein was detected and, the N-terminal amino-acid sequence of the band was determined with a protein sequencer ABI477A (ABI Co., Ltd).

The N-terminal amino-acid sequence of the ocapsid protein was SGKDDPDPIV for the Na1 strain and AGHEEPDPIV for the Ka1 strain. Based on the determination of the base sequence in step (5) and the N-terminal amino-acid sequence of the capsid protein, the primary structure of the capsid protein was elucidated.

The capsid protein of the BaMMV Na1 strain had an amino-acid SEQ ID No:2: and a base SEQ ID No:1: in the SEQUENCE LISTING.

The capsid protein of the BaMMV Ka1 strain had an amino-acid SEQ ID No:4: and a base SEQ ID No:3: in the SEQUENCE LISTING.

The capsid protein of BaYMV (strain type II-1) was isolated according to the above-identified reference Kashiwazaki et al., Journal of General Virology, 70, 3015-3023(1989) and descritpion herein. The capsid protein of the BaYMV II-1 strain had an amino-acid SEQ ID No:6: and a base SEQ ID No:5: in the SEQUENCE LISTING.

### Embodiment

### 1. Construction of Capsid Protein Gene Originated from a Specific Region Coding Capsid Proteins of BaMMV Na1 and BsaMMV Ka1

Using plasmids pBYT10, Na1SK2, and Ka1SK11 including specific regions for coding the capsid proteins of cDNA prepared from the BaYMV, BaMMV-Na1, and BaMMV Ka1 genomes, subcloning and recombination of the specific regions for coding the capsid proteins as separate genes were performed according to the polymerase chain reaction. Preparation methods of the plasmids pBYT10, Na1SK2, and Ka1SK11 have already been published in Kashiwazaki et al., Journal of General Virology, 70, 3015-3023(1989), Kashiwazaki et al., Journal of General Virology, 73, 2173-2181(1992), and a specification of Japanese Patent Kokai Hei 4-180624.

Primer DNAs necessary for the polymerase chain reaction were synthesized with an Applied Biosystems 391 DNA synthesizer, PCR-MATETM EP (ABI Co., Ltd). Structures of the synthesized primer DNAs are shown below:
(1) For BaYMV N-terminal: AAGAATTCATGGCTGCTGATCCTCTCACTGA (SEQ ID NO:7:)
(2) For BaYMV C-terminal: GTGAATTCGATGGTTTAGGTTAGTTCTGGGT (SEQ ID NO:8:)
(3) For BaMMV Na1 N terminal:
   GTGAATTCATGTCAGGAAAAGATGATCCAGA (SEQ ID NO:9:)
(4) For BaMMV Na1 strain C terminal:
   ACGAATTCCTACCGGAGAAGCGCCTCGTGACC (SEQ ID NO:10:)
(5) For BaMMV Ka1 strain N terminal:
   GTGAATTCATGGCAGGACACGAAGAACCAGA (SEQ ID NO:11:)
The primer DNA (4) is also used for the C terminal of the BaMMV Ka1 strain since the capsid proteins of BaMMV Na1 and BaMMV Ka1 strains have the same C-terminal gene structure.

The primer DNA had eight bases including GAATTC, a site of digestion with a restriction enzyme EcoRI, at its 5' terminal to allow a capsid protein gene amplified by the PCR to function as an EcoRI segment. The DNA primer for the N-terminal of the capsid protein gene had a translation starting codon ATG downstream of the EcoRI site to allow a specific region to code the capsid protein to function as a separate structural gene after the PCR. The primer DNA was digested and purified with an oligonucleotide purification column (Applied Biosystem Co., Ltd.) according to the protocol attached. Each primer DNA thus purified was stored in a TE buffer (10 mM Tris-HCl buffer at pH 7.5 and 1 mM EDTA) at -20°C.

The polymerase chain reaction was performed in combinations below with a commercially available PCR kit, GeneAMP: PCR Regent Kit with AmpliTaq DNA polymerase (Perkin Elmer Cetus Co., Ltd.).
(1) BaYMV capsid protein gene, Template: EcoRI-digested pBYT10, Primer DNAs (1) and (2)
(2) BaMMV Na1 capsid protein gene, Template:
   EcoRI-digested Na1SK2, Primer DNAs (3) and (4)
(3) BaMMV Ka1 capsid protein gene, Template:
   EcoRI-digested Ka1SK11, Primer DNAs (4) and (5)
In order to amplify the DNA fragments of invest, a cycle of denaturation reaction at 94 °C for one minute, annealing reaction at 55°C for two minutes, and polymerase reaction PCR at 72°C for three minutes was repeated twenty five times with the temperature controlled using a programmable thermal controller (MJ Research Inc). Digestion of the PCR product with EcoRI and electrophoresis with 0.8% agarose gel resulted in detection of DNA segments of approximately 900 bps were obtained for the template pBYT10 and DNA segments of approximately 750 bps for the templates Na1SK2 and Ka1SK11, which matched with the expected sizes of the virus capsid protein genes.

EcoRI fragments detected as above were extracted from the gel with phenol and chloroform and stored in a TE buffer at -20°C. Each capsid protein gene thus obtained had an EcoRI site at its 5' terminal, a translation starting codon ATG, a specific region for coding the virus cDNA-originated capsid protein, a translation stop codon, and an EcoRI site at its 3' terminal. This was a separate structural gene working as an EcoRI fragment (see Fig. 1).

2. Construction of Capsid Protein Gene-Expressing Plasmid
in Escherichia coli

The capsid protein gene-expressing plasmid was constructed for the purpose of evaluating the functions of each capsid protein gene constructed in Step 1 described above. The Escherichia coli-expressing vector used was pKK223-3. As described previously, the addition of IPTG (isopropyl-β-D-galactopyranoside) to a culture medium activates a tac promoter and a ribosome binding site on the vector pKK223-3 to allow a gene inserted into an EcoRI site on pKK223-3 to be expressed in Escherichia coli. A mixture of 1 µl pKK223-3 (0.02 µg/µl ) digested with EcoRI and dephosphorylated, 5 µl of each capsid protein gene solution (0.02 µg/µl ), and 48 µl of a solution A and 6 µl of a solution B of a DNA ligation kit (Takara Shuzo Co., Ltd.) was ligated at 16°C for two hours.

The ligated reaction solution thus obtained was mixed on ice with 200 µl of a suspension containing competent cells of E. coli XL1-Blue (Toyobo Co., Ltd.) previously prepared, and left standing for thirty minutes. After being incubated at 42°C for thirty seconds, the solution was immediately cooled with ice, and incubated with 0.9 ml of an LB culture medium at 37°C. After one hour, the incubated solution was spread out on plates of an LB agar medium containing 75 µg/ml ampicillin, and cultured overnight at 37°C. The plasmid was prepared from the colony by means of mini-screening and assayed for a target capsid protein gene. According to the above procedures, transformed Escherichia coli having plasmids pSBG202, pSBG211, and pSBG213, each including the corresponding capsid protein gene, were prepared (see Fig. 1). The plasmids pSBG202, pSBG211, and pSBG213 respectively included a BaYMV-originated capsid protein gene, BaMMV Ka1-originated capsid protein gene, and BaMMV Na1-originated capsid protein gene.

3. Expression of Capsid Protein Gene in Escherichia coli

Each capsid protein was produced with the corresponding capsid protein gene-expressing plasmid constructed in Step 2 described above. Four different types of plasmid-containing Escherichia coli, that is, E. coli XL-Blue/pSBG202, E. coli XL-Blue/pSBG211, E. coli XL-Blue/pSBG213, and E. coli XL-Blue/pKK223-3 (control) were pre-incubated overnight on an LB culture medium at 37°C under shaking of 120 rpm/min. Each pre-incubated culture (1 ml) was mixed with 50 ml of a fresh LB liquid medium and incubated under the same conditions as above. IPTG was added to the culture to make the final concentration of the culture equal to 1 mM for the absorbance of 0.4 at 600 nm. After two-hour incubation period, 200 µl of each culture were placed in four Eppendolf tubes and centrifuged at 4 °C and 10,000 x g for two minutes. E. coli samples thus obtained were frozen at -70°C.

Each frozen E. coli sample was suspended in 100 µl of a sample buffer containing 62.6 mM Tris-HCl buffer (pH 6.8), 2% SDS, 10% glycerol, 0.001% Bromophenol Blue, and 1% 2-mercaptoethanol, and incubated at 100°C for two minutes. SDS-PAGE was performed for 10 µl of the sample at a gel concentration of 12% according to Laemmli's method (Laemmli U.K., Nature, 227, 680-685(1970)).

For the detection of the capsid protein with an antibody, the protein in the gel is transcribed onto a PVDF membrane by western blotting. The membrane and the blotting device used for electro-blotting were Immobilon P (Millipore Corp.) and Polyblot (ABN Inc.), respectively. Conditions of blotting followed the standard procedure of ABN Inc. The membrane with the blotted protein was washed in a TBS buffer (20 mM Tris-HCl buffer (pH 7.5) and 137 mM NaCl), and shaken for one hour in a TBS buffer containing 5% bovine serum albumin (Shigma Co., Ltd.). The membrane was further shaken in a TBS buffer containing anti-BaYMV (BaMMV-Na1 or BaMMV Ka1) γ-globulin (2 µg/ml) for two hours, so that the corresponding antibody was bound to the ocapsid protein. γ-Globulin non-specifically adsorbed was removed by washing the membrane three times with a TBST buffer (TBS buffer containing 0.1%(v/v) Tween 20) three times.

A 0.1 mg/ml solution of alkali phosphatase-labelled anti-lapin IgG (Kirkegaard & Perry Laboratories Inc.) used as a secondary antibody was diluted 1,000 times with a TBS buffer. γ-Globulin bound to the capsid protein was further bound to alkali phosphatase-labelled anti-rabbit IgG by shaking the membrane in the diluted solution. The membrane was first washed three times with a TBST buffer to remove the excess alkali phosphatase-labelled anti-rabbit IgG, and then shaken in a color-producing agent containing 0.1M diethanolamine (pH 9.5), 0.3 mg/ml nitroblue tetrazolium, 0.15 mg/ml 5-bromo-4-chloro-3-indolyl phosphate, and 5 mM MgCl₂ for three minutes for the detection of the capsid protein.

Figs. 2A and 2B schematically show electrophoretic migration of the capsid proteins produced in Escherichia coli. Fig. 2A and Fig. 2B respectively show the detection of the capsid proteins with an anti-BaYMV IgG and with an anti-BaMMV IgG. In the drawings, lanes 1 through 7 represent BaYMV (0.1 microgram), BaMMV-Ka1 (0.1 microgram), BaMMV-Na1 (0.1 microgram), XL-1 Blue/pKK223-3, XL-1 Blue/pSB G202, XL-1 Blue/pSB G211, and XL-1 Blue/pSB G213, respectively.

As shown in Figs. 2A and 2B, this resulted in clear protein bands reactive to antibodies originated from the respective capsid protein genes constructed in the above manner. The molecular weight of the detected protein coincided with the molecular weight of the corresponding virus-originated capsid protein.

The protein produced by the BaYMV-originated capsid protein gene reacted with the antibody to the BaYMV capsid protein while not reacting with the antibodies to the BaMMV-Na1 capsid protein and BaMMV-Ka1 capsid protein. In a similar manner, the protein produced by the BaMMV-Na1 or -Ka1-originated capsid protein gene reacted with the antibody to the BaMMV-Na1 or -Ka1 capsid protein while not reacting with the antibody to the BaYMV capsid protein. Such antibody response was also found for each virus capsid protein used as a control.

These results clearly show that each capsid protein gene constructed in the above manner functions to produce a corresponding capsid protein, which is immunologically identical with each virus capsid protein. In the above experiments, several bands not ascribed to the capsid proteins were found in both the test sample with the ocapsid protein gene and the control without the capsid protein gene. This is thereby attributable to the non-specific bonding of the polyclonal antibody with E. coli-originated proteins.

### 4. Construction of Capsid Protein Gene-Expressing Plasmid in Barley Cells

In order to utilize each capsid protein gene constructed as above to produce yellow mosaic disease-resistant barley, it is necessary to make the capsid protein gene expressed in barley cells to produce the corresponding capsid protein. A plasmid pCaMVCN (Pharmacia Corp.) generally used as a control vector for the transduction of a plant gene is used herein to express the capsid protein gene in barley cells. As described previously, this plasmid is constructed to allow the expression of a CAT gene through functions of a 35S promoter and an NOS terminater. The inventors have found that replacement of the CAT gene in the plasmid pCaMVCN with each capsid protein gene allows the expression of the capsid protein gene in barley cells.

After the plasmid pCaMVCN was digested with PstI, vector-originated DNA fragments were separated from DNA fragments including a CAT gene region through agarose gel electrophoresis, and then extracted from the gel.

The vector-originated DNA fragments extracted were treated with a DNA blunting kit (Takara Shuzo Co., Ltd.) containing T4 DNA polymerase as a primary constituent to blunt the PstI-digested terminals. In a similar manner, DNA fragments of each capsid protein gene having EcoRI-digested terminals were blunted. Each capsid protein gene-expressing plasmid was constructed by binding each capsid protein gene thus blunted to the plasmid pCaMVCN without the CAT gene region according to the method described in Step 2. Fig. 3 schematically shows the construction of plasmids making capsid protein genes expressed in barley cells. The following are relations between the plasmid constructed and each virus capsid protein:
pSBG 204: including BaYMV-originated capsid protein gene
pSBG 215: including BaMMV-Ka1-originated capsid protein gene
pSBG 217: including BaMMV-Na1-originated capsid protein gene
It is expected that each capsid protein gene on the plasmid utilizes a 35S promoter of a cauliflower mosaic virus to be expressed in barley cells so as to produce the corresponding capsid protein.

### 5. Expression of Capsid Protein Gene in Barley Cells

Each capsid protein gene-expressing plasmid constructed in Step 4 was assayed for the expression of each capsid protein gene in barley cells to produce the corresponding capsid protein. Each ocapsid protein gene-expressing plasmid was transduced into barley protoplasts by electroporation. The protoplasts used herein was prepared according to the following steps. The barley cells used were Hordeum vulgare immature embryo-originated R215S cells stored at the Plant Engineering Laboratory of Sapporo Breweries Ltd. Two grams of the R215S cells were suspended in 20 ml of an enzyme solution (pH 5.8) containing 2% Cellulase RS (Yakult Honsha Co., Ltd.), 0.1% pectolyase Y-23 (Morishin Seiyaku Co., Ltd.), 0.1% casamino acid, 5 mM MES, and 0.6M mannitol, and shaken at 20°C and 40rpm for two hours. The suspension was filtered through a mesh having a diameter of 26 µm and centrifuged at 20°C and 500 x g to give protoplasts. The protoplasts thus obtained were washed centrifugally with a cleaning solution (pH 5.8) containing 0.6M mannitol and 5 mM MES under the same centrifuging conditions, suspended in a protoplast buffer (pH 5.8) containing 0.6M mannitol, 70 mM KCl, 5 mM MgCl₂, and 5 mM MES to have a predetermined cell density, 2.5 x 10⁶ protoplasts/ml, and left standing in ice for five minutes.

An electroporation apparatus, Gene Pulser (Biorad Inc.) was used for transduction of the gene. The icy cold protoplast solution (800 µl) prepared as above was mixed with 8 µl of a plasmid including a hygromycin-resistant gene pSBG102 (Kaneko et al., Barley Genetics VI volume I, pp.231-233(1991)) solution and the BaYMV capsid protein gene and 8 µl of a pSBG204 solution (1 µg DNA/µl), and placed in a cuvette for electroporation under conditions of 120 V and 500 µF.

The protoplast solution treated by electroporation was mixed with a Kao's medium including 2.5% Sea-plaque agarose (FMC) and 0.6M maltose (Kao et al., Planta 126: 105-110(1975)) for nurse culture. Callus of approximately 0.5 mm in diameter were placed in a Kao's agar medium containing 20 µg/ml hygromycin for selection of hygromycin-resistant callus. This selection step was repeated twice. DNA was extracted from the hygromycin-resistant callus finally selected, and callus with the BaYMV capsid protein gene transduced therein were identified by southern hybridization (Maniatis et al., Molecular Cloning: A Laboratory Manual (1982)).

The callus (1 µg) were frozen with liquid nitrogen, crushed to powder in liquid nitrogen with a blender, and mixed and stirred by a blender with 20 ml of a solution containing 4M guanidine thiocyanate. 0.1M Tris-HCl buffer (pH 7.5), 0.5% Sarkosyl, 0.1% 2-mercaptoethanol and 20 ml of a phenol-chloroform 1:1 solution. The mixed solution placed in a test tube was treated with phenol several times. A water phase of the solution was precipitated with ethanol and subsequently dissolved in a TE buffer. After electrophoresis with formalin-containing agarose, the sample solution (20 µg ) was transcribed onto a nylon membrane Hybond N⁺ (Amersham Co., Ltd.). Expression of mRNA originated from the BaYMV capsid protein gene was assayed by northern blotting (Maniatis et al., Molecular Cloning: A Laboratory Manual(1982)). Using as a template a BaYMV capsid protein gene DNA obtained by digestion of pSBG202 with EcoRI, a radioactive probe was prepared using 1MBq [α-³²P]dCTP with a Multiprime DNA Labelling Kit (Amasham Co., Ltd.) according to a standard procedure of Amasham. After pre-hybridiation and hybridization with a hybridization buffer (6 xSSC, 1% SDS, 0.1 mg/ml bovine thymus DNA) at 60°C the filter was washed with a wash solution (1 xSSC, 0.5% SDS) at 68°C, and then dried. Autoradiography was performed for the filter with an X-ray film.

The cultured cells (1 g) were mixed with 500 µl of the sample buffer, ground in an icy-cold mortar, and centrifuged in a test tube at 10,000 xg and 4°C for thirty minutes. A supernatant sample (20 µl) separated from the centrifuged solution was treated by SDS-PAGE, western blotting, and then with a primary antibody and with a secondary antibody as described in detail in Step 3. The secondary antibody used here was protein A (Amasham Co., Ltd.) labelled with radioactive iodine ¹²⁵I, which was reacted with the sample at a concentration of 0.1 MBq/ml. The membrane finally obtained was dried, and autoradiography was performed with an X-ray film to detect the capsid protein. Autoradiography revealed signals attributed to the BaYMV capsid protein gene as shown in Figs. 4A and 4B. The molecular weight of the detected capsid protein coincided with the molecular weight of the BaYMV capsid protein.

Figs. 4A and 4B schematically show the electrophoretic migration of the capsid proteins produced in barley cells. Fig. 4A shows the expression of capsid protein genes and Fig. 4B shows the ability to produce capsid proteins. In Fig. 4A, lanes 1 through 3 respectively represent a control, a pSB G204 transformant-originated RNA (RNase processed), and a pSB G204 transformant-originated RNA. In Fig. 4B, lanes 1 through 3 respectively denote a control (R215S cell), R215S cell + BaYMV (10 ng), and a pSB G204 transformant.

In the above experiments, several signals not ascribed to the capsid proteins were observed in both the test sample with the capsid protein gene and the control without the capsid protein gene. This is thereby attributable to the non-specific binding of the polyclonal antibody with proteins originated from the cultured cells.

The results clearly show that the BaYMV capsid protein gene constructed in the above manner functions normally in barley cells to produce a capsid protein, which is immunologically identical with the BaYMV capsid protein.

The BaMMV capsid protein gene constructed in a similar manner also functions normally in barley cells to produce a capsid protein, which is immunologically identical with the BaMMV capsid protein.

These facts show that the BaYMV and BaMMV capsid protein genes constructed as above are essential for the production of yellow mosaic disease-resistant barley.

It is clearly understood that the above embodiment is only illustrative and not restrictive in any sense since the invention may be embodied in other forms without departing from the scope or spirit of the essential characteristics thereof. The spirit and scope of the present invention is limited only by the terms of the appended claims.

## Claims

1. A method of preparing a yellow mosaic disease-resistant barley, said method comprising the steps of:
(a) isolating a gene including a specific gene region coding a capsid protein, as a capsid protein gene, from cDNA of a barley yellow mosaic virus genome;
(b) conjugating said capsid protein gene with a plasmid vector or rearranging said capsid protein gene and then conjugating the rearranged gene with a plasmid vector, in order to construct a vector DNA having an ability to transform barley cells;
(c) transforming the barley cells with said vector DNA; and
(d) making the transformed barley cells regenerated in a barley plant, so as to make the capsid protein originated from said capsid protein gene expressed in said barley plant to prepare said yellow mosaic disease-resistant barley.

2. The method according to Claim 1, wherein said capsid protein gene comprises a gene isolated from a type strain II-1 of a barley yellow mosaic virus.

3. A method of preparing a yellow mosaic disease-resistant barley, said method comprising the steps of:
(a) isolating a gene including a specific gene region coding a capsid protein, as a capsid protein gene, from cDNA of a barley mild mosaic virus genome;
(b) conjugating said capsid protein gene with a plasmid vector or rearranging said capsid protein gene and then conjugating the rearranged gene with a plasmid vector, in order to construct a vector DNA having an ability to transform barley cells;
(c) transforming the barley cells with said vector DNA; and
(d) making the transformed barley cells regenerated in a barley plant, so as to make the outer-coat protein originated from said capsid protein gene expressed in said barley plant to prepare said yellow mosaic disease-resistant barley.

4. The method according to Claim 3, wherein said capsid protein comprises a protein having an antibody response to a capsid protein of an Na1 strain and/or a Ka1 strain of a barley mild mosaic virus.

5. The method according to Claim 3, wherein said capsid protein gene comprises a gene isolated from an Na1 strain or Ka1 strain of a barley mild mosaic virus.

6. A yellow mosaic resistant barley comprising in its genome a gene including a specific gene region encoding a barley yellow mosaic virus or barley mild mosaic virus capsid protein.

7. The yellow mosaic resistant barley according to claim 6, wherein said gene is derived from a cDNA.

8. The yellow mosaic resistant barley according to claim 6 or 7 which is obtainable according to a method of any one of claims 1 to 5.

9. The yellow mosaic resistant barley according to any one of claims 6 to 8, wherein said gene is located in the chromosomal DNA.


SEQUENCE LISTING

(1) GENERAL INFORMATION:

(i) APPLICANT:
(A) NAME: Director of National Agriculture Research
Center, Ministry of Agriculture, Forestry
and Fisheries
(B) STREET: 1-1, Kannondai 3-chome, Tsukuba-shi
(C) CITY: Ibaraki-ken
(E) COUNTRY: Japan
(F) POSTAL CODE (ZIP): 305

(A) NAME: Sapporo Breweries Ltd.
(B) STREET: 10-1, Ginza 7-chome
(C) CITY: Chuo-ku, Tokyo
(E) COUNTRY: Japan
(F) POSTAL CODE (ZIP): 104

(ii) TITLE OF INVENTION: PREPARATION OF YELLOW MOSAIC
DISEASE-RESISTANT BARLEY

(iii) NUMBER OF SEQUENCES: 11

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(vi) PRIOR APPLICATION DATA:
(A) APPLICATION NUMBER: JP 91933/1993
(B) FILING DATE: 29-MAR-1993

(2) INFORMATION FOR SEQ ID NO: 1:

(i) SEQUENCE CHARACTERISTICS:
(A) LENGTH: 753 base pairs
(B) TYPE: nucleic acid
(C) STRANDEDNESS: double
(D) TOPOLOGY: linear

(ii) MOLECULE TYPE: cDNA

(iii) HYPOTHETICAL: NO

(iii) ANTI-SENSE: NO

(ix) FEATURE:
(A) NAME/KEY: CDS
(B) LOCATION: 1..753

(xi) SEQUENCE DESCRIPTION: SEQ ID NO: 1:

TCA GGA AAA GAT GAT CCA GAT CCC ATT GTT CCT CCA GTT TCT GAC ACT 48
Ser Gly Lys Asp Asp Pro Asp Pro Ile Val Pro Pro Val Ser Asp Thr
1               5                   10                  15

GAT CTC ACA AAC ATG GCT GCA GCA CCA CCC GAC AAC AGA AGG TCG CGC 96
Asp Leu Thr Asn Met Ala Ala Ala Pro Pro Asp Asn Arg Arg Ser Arg
            20                  25                  30

GCC GTC GTC CCC CGT GGG ACC AGT GAC TGG AAT TTG CCT GAA CCC AAG 144
Ala Val Val Pro Arg Gly Thr Ser Asp Trp Asn Leu Pro Glu Pro Lys
        35                  40                  45

ATG CGA ATG CTA GGT TTC AAG TCC AAA ATC AAC ATA GAA ACG CTC GCC 192
Met Arg Met Leu Gly Phe Lys Ser Lys Ile Asn Ile Glu Thr Leu Ala
    50                  55                  60

GAT GTC CCC GAA GGG TAT ATG AAT ACC TTT GCG TCC GTT GCA ACT GAA 240
Asp Val Pro Glu Gly Tyr Met Asn Thr Phe Ala Ser Val Ala Thr Glu
65                  70                  75                  80

ACG CAA CGT AGG AAA TGG GAA GAG GCC ACG CGT GGT GAT TTT GGC ATC 288
Thr Gln Arg Arg Lys Trp Glu Glu Ala Thr Arg Gly Asp Phe Gly Ile
                85                  90                  95

ACC GAC AAC GAG AAG TGG GAA AAG CTG CTC ATT GCT GCC TGC ATA TAT 336
Thr Asp Asn Glu Lys Trp Glu Lys Leu Leu Ile Ala Ala Cys Ile Tyr
            100                 105                 110

TTC GCA GAC AAT GGC ACT TCA CCA AAC TTC GAT GAG GAG CTC ACT ATG 384
Phe Ala Asp Asn Gly Thr Ser Pro Asn Phe Asp Glu Glu Leu Thr Met
        115                 120                 125

GAA GTC AAT GGC GGT CTC AAC TCC ATC AAA GAG TAT CCC GTC CGC CCT 432
Glu Val Asn Gly Gly Leu Asn Ser Ile Lys Glu Tyr Pro Val Arg Pro
    130                 135                 140

TTT GTT GTC AGG GCC AAA AAG ATC TCC ACA CTG CGC AGG ATC TTT CGC 480
Phe Val Val Arg Ala Lys Lys Ile Ser Thr Leu Arg Arg Ile Phe Arg
145                 150                 155                 160

TGT TAC TCC ATT GAG ACG AAA CTA ATG TTC GTG AAG CTG AGG CGC GTA 528
Cys Tyr Ser Ile Glu Thr Lys Leu Met Phe Val Lys Leu Arg Arg Val
                165                 170                 175

CCA CAA TGG GCT ATA AAG CAC GGG TGC CTT GAT GAA ATC GTC TTC GAT 576
Pro Gln Trp Ala Ile Lys His Gly Cys Leu Asp Glu Ile Val Phe Asp
            180                 185                 190

TTC ATG ATC CCA GAC CAG TTC ACT TCC AGA ACT GCA CTC GAA ACA CTG 624
Phe Met Ile Pro Asp Gln Phe Thr Ser Arg Thr Ala Leu Glu Thr Leu
        195                 200                 205

AAG CAG ACC AAG CTT GCT GCC ATT GGT GTT GGC ACA AGC AAT TCT CTC 672
Lys Gln Thr Lys Leu Ala Ala Ile Gly Val Gly Thr Ser Asn Ser Leu
    210                 215                 220

CTC ACC TCC GAA CAG ACG AAC ATG AGA ACC ACT GAA ACC CGA AGG CGC     720
Leu Thr Ser Glu Gln Thr Asn Met Arg Thr Thr Glu Thr Arg Arg Arg
225                 230                 235                 240

AAC GAC TAC GAC GGT CAC GAG GCG CTT CTC CGG                         753
Asn Asp Tyr Asp Gly His Glu Ala Leu Leu Arg
                245                 250

(2) INFORMATION FOR SEQ ID NO: 2:

    (i) SEQUENCE CHARACTERISTICS:
        (A) LENGTH: 251 amino acids
        (B) TYPE: amino acid
        (D) TOPOLOGY: linear

    (ii) MOLECULE TYPE: protein

    (xi) SEQUENCE DESCRIPTION: SEQ ID NO: 2:

Ser Gly Lys Asp Asp Pro Asp Pro Ile Val Pro Pro Val Ser Asp Thr
  1               5                  10                  15

Asp Leu Thr Asn Met Ala Ala Ala Pro Pro Asp Asn Arg Arg Ser Arg
             20                  25                  30

Ala Val Val Pro Arg Gly Thr Ser Asp Trp Asn Leu Pro Glu Pro Lys
         35                  40                  45

Met Arg Met Leu Gly Phe Lys Ser Lys Ile Asn Ile Glu Thr Leu Ala
     50                  55                  60

Asp Val Pro Glu Gly Tyr Met Asn Thr Phe Ala Ser Val Ala Thr Glu
 65                  70                  75                  80

Thr Gln Arg Arg Lys Trp Glu Glu Ala Thr Arg Gly Asp Phe Gly Ile
                 85                  90                  95

Thr Asp Asn Glu Lys Trp Glu Lys Leu Leu Ile Ala Ala Cys Ile Tyr
            100                 105                 110

Phe Ala Asp Asn Gly Thr Ser Pro Asn Phe Asp Glu Glu Leu Thr Met
        115                 120                 125

Glu Val Asn Gly Gly Leu Asn Ser Ile Lys Glu Tyr Pro Val Arg Pro
    130                 135                 140

Phe Val Val Arg Ala Lys Lys Ile Ser Thr Leu Arg Arg Ile Phe Arg
145                 150                 155                 160

Cys Tyr Ser Ile Glu Thr Lys Leu Met Phe Val Lys Leu Arg Arg Val
                165                 170                 175

Pro Gln Trp Ala Ile Lys His Gly Cys Leu Asp Glu Ile Val Phe Asp
            180                 185                 190

Phe Met Ile Pro Asp Gln Phe Thr Ser Arg Thr Ala Leu Glu Thr Leu
        195                 200                 205

Lys Gln Thr Lys Leu Ala Ala Ile Gly Val Gly Thr Ser Asn Ser Leu
    210                 215                 220

Leu Thr Ser Glu Gln Thr Asn Met Arg Thr Thr Glu Thr Arg Arg Arg
225                 230                 235                 240

Asn Asp Tyr Asp Gly His Glu Ala Leu Leu Arg
                245                 250

(2) INFORMATION FOR SEQ ID NO: 3:

     (i) SEQUENCE CHARACTERISTICS:
          (A) LENGTH: 753 base pairs
          (B) TYPE: nucleic acid
          (C) STRANDEDNESS: double
          (D) TOPOLOGY: linear

    (ii) MOLECULE TYPE: cDNA

   (iii) HYPOTHETICAL: NO

   (iii) ANTI-SENSE: NO

    (ix) FEATURE:
          (A) NAME/KEY: CDS
          (B) LOCATION: 1..753

    (xi) SEQUENCE DESCRIPTION: SEQ ID NO: 3:

GCA GGA CAC GAA GAA CCA GAC CCC ATC GTT CCC CCA GTT CCT GAC ACC      48
Ala Gly His Glu Glu Pro Asp Pro Ile Val Pro Pro Val Pro Asp Thr
  1               5                  10                  15

GAT CTA ACG AAC ATG GCT GCC GCA CCA CCC GAC AAC AGA AAG TCG CGC      96
Asp Leu Thr Asn Met Ala Ala Ala Pro Pro Asp Asn Arg Lys Ser Arg
             20                  25                  30

GCC GTC ATC CCC CGT GGG ACC AGT GAC TGG AGC CTG CCT GAA CCC AAA     144
Ala Val Ile Pro Arg Gly Thr Ser Asp Trp Ser Leu Pro Glu Pro Lys
         35                  40                  45

ATG CGA ACA CTG GGT TTC AAG TCC AAG ATC AAC ATC GAA ACA CTT GCC     192
Met Arg Thr Leu Gly Phe Lys Ser Lys Ile Asn Ile Glu Thr Leu Ala
     50                  55                  60

AAT GTC CCC GAC GGG TAT ATG AAC ACC TTT GCA TCC GTT GCA ACT GAA     240
Asn Val Pro Asp Gly Tyr Met Asn Thr Phe Ala Ser Val Ala Thr Glu
 65                  70                  75                  80

TCG CAA CGT AGG AAA TGG GAG GAG GCC GCA CGT GGC GAT TTC GGC ATC     288
Ser Gln Arg Arg Lys Trp Glu Glu Ala Ala Arg Gly Asp Phe Gly Ile
                 85                  90                  95

ACT GAC GAT GAG AAA TGG GAG AAG TTG CTC ATT GCT GCT TGT ATA TAT     336
Thr Asp Asp Glu Lys Trp Glu Lys Leu Leu Ile Ala Ala Cys Ile Tyr
            100                 105                 110

TTC GCA GAC AAT GGC ACC TCA CCG AAT TTC GAC GAG GAA CTC ACT ATG     384
Phe Ala Asp Asn Gly Thr Ser Pro Asn Phe Asp Glu Glu Leu Thr Met
        115                 120                 125

GAG GTT AAT GGC GGT CTC AAC TCC ATC AAA GAG TAC CCC GTT CGC CCC     432
Glu Val Asn Gly Gly Leu Asn Ser Ile Lys Glu Tyr Pro Val Arg Pro
    130                 135                 140

TTT GTC GTT AGA GCC AAA AAG ATT TCC ACG CTG CGC AGA ATC TTT CGC     480
Phe Val Val Arg Ala Lys Lys Ile Ser Thr Leu Arg Arg Ile Phe Arg
145                 150                 155                 160

TGT TAC TCC ATC GAA ACG AAG CTC ATG TTC GTG AAG CTG AGG CGC GTG     528
Cys Tyr Ser Ile Glu Thr Lys Leu Met Phe Val Lys Leu Arg Arg Val
                165                 170                 175

CCA CAC TGG GCC ATA AAG CAC GGA TGC CTC GAT GAG ATC GTC TTT GAT     576
Pro His Trp Ala Ile Lys His Gly Cys Leu Asp Glu Ile Val Phe Asp
            180                 185                 190

TTC ATG ATC CCC GAC CAG TTC ACC TCT AGG ACT GCG CTT GAA ACA CTG     624
Phe Met Ile Pro Asp Gln Phe Thr Ser Arg Thr Ala Leu Glu Thr Leu
        195                 200                 205

AAG CAG ACC AAA CTG GCT GCC ATT GGT GTT GGC ACA AGC AAT TCT CTC     672
Lys Gln Thr Lys Leu Ala Ala Ile Gly Val Gly Thr Ser Asn Ser Leu
    210                 215                 220

CTC ACC TCC GAG CAG ACG AAC ATG AGG ACC ACT GAA ACC CGA CGG CGC     720
Leu Thr Ser Glu Gln Thr Asn Met Arg Thr Thr Glu Thr Arg Arg Arg
225                 230                 235                 240

AAT GAC TAC GAT GGT CAC GAG GCG CTC CTC CGG                         753
Asn Asp Tyr Asp Gly His Glu Ala Leu Leu Arg
                245                 250

(2) INFORMATION FOR SEQ ID NO: 4:

     (i) SEQUENCE CHARACTERISTICS:
          (A) LENGTH: 251 amino acids
          (B) TYPE: amino acid
          (D) TOPOLOGY: linear

    (ii) MOLECULE TYPE: protein

    (xi) SEQUENCE DESCRIPTION: SEQ ID NO: 4:
   Ala Gly His Glu Glu Pro Asp Pro Ile Val Pro Pro Val Pro Asp Thr
     1               5                  10                  15
   Asp Leu Thr Asn Met Ala Ala Ala Pro Pro Asp Asn Arg Lys Ser Arg
                20                  25                  30
   Ala Val Ile Pro Arg Gly Thr Ser Asp Trp Ser Leu Pro Glu Pro Lys
            35                  40                  45
   Met Arg Thr Leu Gly Phe Lys Ser Lys Ile Asn Ile Glu Thr Leu Ala
        50                  55                  60
   Asn Val Pro Asp Gly Tyr Met Asn Thr Phe Ala Ser Val Ala Thr Glu
   65 70 75 80
   Ser Gln Arg Arg Lys Trp Glu Glu Ala Ala Arg Gly Asp Phe Gly Ile
   85 90 95
   Thr Asp Asp Glu Lys Trp Glu Lys Leu Leu Ile Ala Ala Cys Ile Tyr
   100 105 110
   Phe Ala Asp Asn Gly Thr Ser Pro Asn Phe Asp Glu Glu Leu Thr Met
   115 120 125
   Glu Val Asn Gly Gly Leu Asn Ser Ile Lys Glu Tyr Pro Val Arg Pro
   130 135 140
   Phe Val Val Arg Ala Lys Lys Ile Ser Thr Leu Arg Arg Ile Phe Arg
   145 150 155 160
   Cys Tyr Ser Ile Glu Thr Lys Leu Met Phe Val Lys Leu Arg Arg Val
   165 170 175
   Pro His Trp Ala Ile Lys His Gly Cys Leu Asp Glu Ile Val Phe Asp
   180 185 190
   Phe Met Ile Pro Asp Gln Phe Thr Ser Arg Thr Ala Leu Glu Thr Leu
   195 200 205
   Lys Gln Thr Lys Leu Ala Ala Ile Gly Val Gly Thr Ser Asn Ser Leu
   210 215 220
   Leu Thr Ser Glu Gln Thr Asn Met Arg Thr Thr Glu Thr Arg Arg Arg
   225 230 235 240
   Asn Asp Tyr Asp Gly His Glu Ala Leu Leu Arg
   245 250

(2) INFORMATION FOR SEQ ID NO: 5:

     (i) SEQUENCE CHARACTERISTICS:
          (A) LENGTH: 891 base pairs
          (B) TYPE: nucleic acid
          (C) STRANDEDNESS: double
          (D) TOPOLOGY: linear

    (ii) MOLECULE TYPE: cDNA

   (iii) HYPOTHETICAL: NO

   (iii) ANTI-SENSE.: NO

    (ix) FEATURE:
          (A) NAME/KEY: CDS
          (B) LOCATION: 1..891

(xi) SEQUENCE DESCRIPTION: SEQ ID NO: 5:

GCT GCT GAT CCT CTC ACT GAT GCA CAA AAA GAA GAT GCA CGC ATT GCT 48
Ala Ala Asp Pro Leu Thr Asp Ala Gln Lys Glu Asp Ala Arg Ile Ala
1               5                   10                  15

GCA GCT GAT GGA GCA CGC TTC GAA CTA GCC GAT GCT GAT CGT AGA CGT 96
Ala Ala Asp Gly Ala Arg Phe Glu Leu Ala Asp Ala Asp Arg Arg Arg
            20                  25                  30

AAA GTT GAG GCT GAC CGT GTG GAA GCG GCA AGA GTC AAA AAG GCA GCT 144
Lys Val Glu Ala Asp Arg Val Glu Ala Ala Arg Val Lys Lys Ala Ala
        35                  40                  45

GAT GCT GCA CTA AAA CCA GTT AAC CTC ACA GCA ACA CGC ACG CCC ACA 192
Asp Ala Ala Leu Lys Pro Val Asn Leu Thr Ala Thr Arg Thr Pro Thr
    50                  55                  60

GAA GAT GAT GGC AAA CTT AAA ACA CCA TCT GGC GCT AGA ATA CCA TCA 240
Glu Asp Asp Gly Lys Leu Lys Thr Pro Ser Gly Ala Arg Ile Pro Ser
65                  70                  75                  80

TCA GCT GCA GAT GGT AAC TGG AGC GTG CCA GCA ACG AAG CAA GTT AAC 288
Ser Ala Ala Asp Gly Asn Trp Ser Val Pro Ala Thr Lys Gln Val Asn
                85                  90                  95

GCT GGT TTG ACA CTC AAA ATT CCC CTG AAT AAG CTC AAA AGT GTA CCT 336
Ala Gly Leu Thr Leu Lys Ile Pro Leu Asn Lys Leu Lys Ser Val Pro
            100                 105                 110

AAG TCT GTT ATG GAG CAT AAC AAC TCA GTC GCA CTC GAA TCT GAG CTA 384
Lys Ser Val Met Glu His Asn Asn Ser Val Ala Leu Glu Ser Glu Leu
        115                 120                 125

AAA GCC TGG ACT GAT GCT GTG CGC ACA AGT TTG GGT ATC ACA ACT GAT 432
Lys Ala Trp Thr Asp Ala Val Arg Thr Ser Leu Gly Ile Thr Thr Asp
    130                 135                 140

GAG GCA TGG ATC GAT GCC CTC ATT CCC TTC ATC GGA TGG TGC TGC AAC 480
Glu Ala Trp Ile Asp Ala Leu Ile Pro Phe Ile Gly Trp Cys Cys Asn
145                 150                 155                 160

AAT GGC ACC TCA GAC AAG CAT GCT GAG AAT CAA GTA ATG CAA ATT GAC 528
Asn Gly Thr Ser Asp Lys His Ala Glu Asn Gln Val Met Gln Ile Asp
                165                 170                 175

AGC GGA AAG GGA GCT GTT ACA GAG ATG AGT CTG TCC CCA TTT ATT GTT 576
Ser Gly Lys Gly Ala Val Thr Glu Met Ser Leu Ser Pro Phe Ile Val
            180                 185                 190

CAT GCT CGA ATG AAT GGT GGA CTC CGA AGG ATC ATG CGC AAC TAC AGT 624
His Ala Arg Met Asn Gly Gly Leu Arg Arg Ile Met Arg Asn Tyr Ser
        195                 200                 205

GAT GAA ACC GTC TTA CTC ATC ACA AAC AAC AAA CTA GTT GCA CAT TGG 672
Asp Glu Thr Val Leu Leu Ile Thr Asn Asn Lys Leu Val Ala His Trp
    210                 215                 220

TCA ATG AAG CAT GGC GCT TCT GCA AAC GCA AAA TAC GCT TTC GAT TTC 720
Ser Met Lys His Gly Ala Ser Ala Asn Ala Lys Tyr Ala Phe Asp Phe
225 . 230 235 240

TTC GTC CCA CGA TCA TGG ATG AAC CCA CAG GAT ATC GAA GTG TCA AAG 768
Phe Val Pro Arg Ser Trp Met Asn Pro Gln Asp Ile Glu Val Ser Lys
245 250 255

CAA GCG CGC TTA GCA GCA CTT GGA ACT GGA ACG TAT AAC ACC ATG TTA 816
Gln Ala Arg Leu Ala Ala Leu Gly Thr Gly Thr Tyr Asn Thr Met Leu
260 265 270

ACT TCC GAC ACT ACC AAT CTG CGT AAG ACA ACC AAC CAC AGG GTT CTG 864
Thr Ser Asp Thr Thr Asn Leu Arg Lys Thr Thr Asn His Arg Val Leu
275 280 285

GAC TCA GAT GGA CAC CCA GAA CTA ACC 891
Asp Ser Asp Gly His Pro Glu Leu Thr
290 295

(2) INFORMATION FOR SEQ ID NO: 6:

(i) SEQUENCE CHARACTERISTICS:
(A) LENGTH: 297 amino acids
(B) TYPE: amino acid
(D) TOPOLOGY: linear

(ii) MOLECULE TYPE: protein

(xi) SEQUENCE DESCRIPTION: SEQ ID NO: 6:

Ala Ala Asp Pro Leu Thr Asp Ala Gln Lys Glu Asp Ala Arg Ile Ala
1               5                   10                  15

Ala Ala Asp Gly Ala Arg Phe Glu Leu Ala Asp Ala Asp Arg Arg Arg
            20                  25                  30

Lys Val Glu Ala Asp Arg Val Glu Ala Ala Arg Val Lys Lys Ala Ala
        35                  40                  45

Asp Ala Ala Leu Lys Pro Val Asn Leu Thr Ala Thr Arg Thr Pro Thr
    50                  55                  60

Glu Asp Asp Gly Lys Leu Lys Thr Pro Ser Gly Ala Arg Ile Pro Ser
65                  70                  75                  80

Ser Ala Ala Asp Gly Asn Trp Ser Val Pro Ala Thr Lys Gln Val Asn
                85                  90                  95

Ala Gly Leu Thr Leu Lys Ile Pro Leu Asn Lys Leu Lys Ser Val Pro
            100                 105                 110

Lys Ser Val Met Glu His Asn Asn Ser Val Ala Leu Glu Ser Glu Leu
        115                 120                 125

Lys Ala Trp Thr Asp Ala Val Arg Thr Ser Leu Gly Ile Thr Thr Asp
    130                 135                 140

Glu Ala Trp Ile Asp Ala Leu Ile Pro Phe Ile Gly Trp Cys Cys Asn
145                 150                 155                 160

Asn Gly Thr Ser Asp Lys His Ala Glu Asn Gln Val Met Gln Ile Asp
                165                 170                 175

Ser Gly Lys Gly Ala Val Thr Glu Met Ser Leu Ser Pro Phe Ile Val
            180                 185                 190

His Ala Arg Met Asn Gly Gly Leu Arg Arg Ile Met Arg Asn Tyr Ser
        195                 200                 205

Asp Glu Thr Val Leu Leu Ile Thr Asn Asn Lys Leu Val Ala His Trp
    210                 215                 220

Ser Met Lys His Gly Ala Ser Ala Asn Ala Lys Tyr Ala Phe Asp Phe
225                 230                 235                 240

Phe Val Pro Arg Ser Trp Met Asn Pro Gln Asp Ile Glu Val Ser Lys
                245                 250                 255

Gln Ala Arg Leu Ala Ala Leu Gly Thr Gly Thr Tyr Asn Thr Met Leu
            260                 265                 270

Thr Ser Asp Thr Thr Asn Leu Arg Lys Thr Thr Asn His Arg Val Leu
        275                 280                 285

Asp Ser Asp Gly His Pro Glu Leu Thr
    290                 295

(2) INFORMATION FOR SEQ ID NO: 7:

     (i) SEQUENCE CHARACTERISTICS:
          (A) LENGTH: 31 base pairs
          (B) TYPE: nucleic acid
          (C) STRANDEDNESS: single
          (D) TOPOLOGY: linear

    (ii) MOLECULE TYPE: cDNA

   (iii) HYPOTHETICAL: NO

   (iii) ANTI-SENSE: NO

    (xi) SEQUENCE DESCRIPTION: SEQ ID NO: 7:

AAGAATTCAT GGCTGCTGAT CCTCTCACTG A                                31

(2) INFORMATION FOR SEQ ID NO: 8:

     (i) SEQUENCE CHARACTERISTICS:
          (A) LENGTH: 31 base pairs
          (B) TYPE: nucleic acid
          (C) STRANDEDNESS: single
          (D) TOPOLOGY: linear

    (ii) MOLECULE TYPE: cDNA

(iii) HYPOTHETICAL: NO

(iii) ANTI-SENSE: NO

(xi) SEQUENCE DESCRIPTION: SEQ ID NO: 8:

GTGATTTCGA TGGTTTAGGT TAGTTCTGGG T 31

(2) INFORMATION FOR SEQ ID NO: 9:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 9:

GTGAATTCAT GTCAGGAAAA GATGATCCAG A 31

(2) INFORMATION FOR SEQ ID NO: 10:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 10:

ACGAATTCCT ACCGGAGAAG CGCCTCGTGA CC 32

(2) INFORMATION FOR SEQ ID NO: 11:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 11:

GTGAATTCAT GGCAGGACAC GAAGAACCAG A 31